(19) Europäisches Patentamt
European Patent Office
Office européen des brevets

(11) EP 1 360 171 B1

(12) **EUROPÄISCHE PATENTSCHRIFT**

(45) Veröffentlichungstag und Bekanntmachung des Hinweises auf die Patenterteilung:
**14.05.2008 Patentblatt 2008/20**

(21) Anmeldenummer: **02714146.4**

(22) Anmeldetag: **05.02.2002**

(51) Int Cl.:
***C07C 261/02*** *(2006.01)* ***C08G 73/06*** *(2006.01)*

(86) Internationale Anmeldenummer:
**PCT/EP2002/001154**

(87) Internationale Veröffentlichungsnummer:
**WO 2002/064548 (22.08.2002 Gazette 2002/34)**

(54) **PREPOLYMER-ZUSAMMENSETZUNGEN AUF BASIS VON NOVOLAK-CYANATEN**

NOVOLAK CYANATE-BASED PREPOLYMER COMPOSITIONS

COMPOSITIONS PREPOLYMERES A BASE DE CYANATES DE NOVOLAQUE

(84) Benannte Vertragsstaaten:
**AT BE CH CY DE DK ES FI FR GB GR IE IT LI LU MC NL PT SE TR**

(30) Priorität: **09.02.2001 EP 01103068**
**14.11.2001 US 331300 P**

(43) Veröffentlichungstag der Anmeldung:
**12.11.2003 Patentblatt 2003/46**

(73) Patentinhaber: **Lonza AG**
**4052 Basel (CH)**

(72) Erfinder:
- **GMÜR, Martin**
  **CH-6314 Unterägeri (CH)**
- **DAUM, Ulrich**
  **CH-4114 Hofstetten (CH)**
- **HEYL-FRANK, Brigitta**
  **CH-3930 Visp (CH)**
- **HANSELMANN, Paul**
  **CH-3902 Glis (CH)**
- **FALCHETTO, Alessandro**
  **I-28845 Domodossola (IT)**

(56) Entgegenhaltungen:
**US-A- 4 094 852** **US-A- 4 713 442**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die Erfindung betrifft Prepolymer-Zusammensetzungen auf Basis von Novolak-Cyanaten.

Novolak-Cyanate sind reaktionsfähige oligomere Verbindungen der allgemeinen Formel

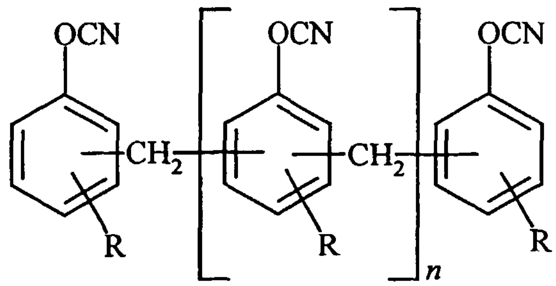

(I),

worin *n* im allgemeinen eine Zahl von 0 bis 20 ist und die Reste R gleich oder verschieden sind und für Wasserstoff oder Methyl stehen. Die Bindungen zu den die Benzolringe verknüpfenden Methylengruppen können grundsätzlich von den ortho- (*o*), meta- (m) oder para- (*p*) Positionen zu den Cyanatgruppen ausgehen und die Reste R können sich in jeder der verbleibenden Positionen befinden. Vorzugsweise erfolgt die Verknüpfung über die ortho- und para-Positionen. Die Verbindungen liegen üblicherweise sowohl als Oligomerengemische (mit verschiedenen Werten von *n*) als auch als Isomerengemische (mit unterschiedlichen Verknüpfungsmustern, vorzugsweise *o* oder *p* für die endständigen und *o*,*o* oder *o*,*p* für die nicht endständigen Benzolringe) vor und sind aus den Kondensationsprodukten von Phenol oder Kresolen mit Formaldehyd durch Umsetzung mit Chlor- oder Bromcyan erhältlich. Die Oligomerengemische werden üblicherweise durch Angabe des Mittelwertes von *n* charakterisiert. Dieser Mittelwert kann - im Gegensatz zu dem Wert von *n* in einem einzelnen Molekül - auch nicht-ganzzahlig sein.

Novolak-Cyanate (I) mit R = Wasserstoff oder R = Methyl sind im Handel erhältlich, beispielsweise unter der Bezeichnung Primaset® von der Firma Lonza AG, Basel, Schweiz.

Die Novolak-Cyanate können thermisch, gegebenenfalls in Gegenwart von Katalysatoren, zu Polytriazinen polymerisiert werden, wobei jeweils drei Cyanatgruppen zu einem 1,3,5-Triazinring cyclotrimerisieren. Diese Cyclotrimerisierung ist stark exotherm.

Wegen der hervorragenden thermischen, mechanischen und elektrischen Eigenschaften von Polytriazinen werden Novolak-Cyanate in zunehmendem Umfang allein oder im Gemisch mit anderen Cyanaten zur Herstellung hochtemperaturbeständiger Formteile eingesetzt.

Um die Verarbeitungseigenschaften zu verbessern und die Exothermie bei der Aushärtung herabzusetzen, ist es für einige Anwendungen zweckmässig, die Cyclotrimerisierung teilweise schon vor der Formgebung durchzuführen, so dass ein - vorzugsweise festes - "Prepolymer" erhalten wird, das noch freie Cyanatgruppen aufweist, gegebenenfalls in organischen Lösungsmitteln löslich ist und nach der Formgebung unter Reaktion der verbliebenen Cyanatgruppen vollends ausgehärtet und vernetzt werden kann. Die Prepolymeren aus dem Stand der Technik weisen jedoch auch negative Eigenschaften auf, indem sie klebrig und weder gut mahlbar noch rieselfähig oder wegen zu starker Vernetzung nicht mehr formbar sind oder nicht die gewünschte Reaktivität aufweisen.

Aufgabe der vorliegenden Erfindung war daher die Bereitstellung von Novolak-Cyanat-Prepolymeren, die bei Normaltemperatur fest und bei höherer Temperatur schmelzbar sind, sich leicht zu einem gut rieselfähigen Pulver mahlen lassen und weder zu reaktiv noch zu reaktionsträge sind. Insbesondere sollten sie bei Normaltemperatur lange Zeit lagerfähig sein, bei 200 °C eine Gelzeit von mindestens 6 Minuten aufweisen, aber nach der Gelbildung rasch vollständig aushärten.

Erfindungsgemäss wird diese Aufgabe durch die Prepolymer-Zusammensetzungen nach Patentanspruch 1 gelöst.

Es wurde gefunden, dass bestimmte Gemische von prepolymerisierten und nicht prepolymerisierten Novolak-Cyanaten, nämlich Gemische aus 30 bis 90 Gewichtsteilen von Prepolymeren aus Novolak-Cyanaten der allgemeinen Formel

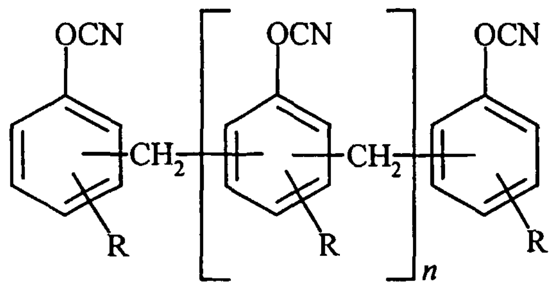

(I),

worin *n* eine Zahl von 0 bis 20 ist und die Reste R für Wasserstoff stehen (Ia), mit 10 bis 70 Gewichtsteilen von Novolak-Cyanaten (I), in denen R jeweils für Methyl steht (Ib), die gewünschten Eigenschaften aufweisen. Die Summe der Gewichtsteile von Prepolymer und Novolak-Cyanat (Ib) beträgt hierbei jeweils 100.

Vorzugsweise enthalten die erfindungsgemässen Zusammensetzungen 50 bis 70 Gewichtsteile Prepolymere aus Novolak-Cyanaten (Ia) und 30 bis 50 Gewichtsteile Novolak-Cyanat (Ib).

Vorzugsweise ist der Mittelwert von *n* in dem Novolak-Cyanat (Ia), aus dem das Prepolymer erhalten wurde, eine Zahl von 0 bis 5.

Besonders gute Resultate werden hierbei mit Prepolymeren erzielt, die einen Brechungsindex $n_D^{98}$ (Brechungsindex für die Na-D-Linie bei 98 ˚C) von mindestens 1,58, vorzugsweise mindestens 1,582 aufweisen.

Ebenfalls bevorzugt sind Prepolymer-Zusammensetzungen, bei denen der Mittelwert von *n* in den Novolak-Cyanaten (Ib) 1 bis 10 beträgt.

In einer bevorzugten Ausführungsform enthalten die erfindungsgemässen Prepolymer-Zusammensetzungen noch zusätzlich bis zu 5 Gew% eines hochdispersen Siliciumdioxids. Als hochdisperses Siliciumdioxid können sowohl pyrogene (z. B. Aerosil®) als auch gefällte Produkte (z. B. Sipernat®) eingesetzt werden.

Besonders bevorzugt sind pyrogene Siliciumdioxide.

Die erfindungsgemässen Prepolymer-Zusammensetzungen eignen sich insbesondere als Harzkomponente in Basismaterialien für Leiterplatten oder kunstharzgebundenen Schleifkörpern.

Die folgenden Beispiele verdeutlichen die Ausführung der Erfindung, ohne dass darin eine Einschränkung zu sehen ist.

Die Bestimmung der Gelzeiten wurde bei 200±1 ˚C analog zu DIN 16 945 mit Gelnorm®-Geräten (Gel Instrumente AG, CH-8800 Thalwil) an Proben von ca. 12 g in herkömmlichen Reagenzgläsern (16×160 mm) durchgeführt. Die Dauer eines Hubzyklus war 10 s.

Zur Bestimmung der Brechnungsindices wurde ein auf 98 ˚C thermostatisiertes Abbé-Refraktometer eingesetzt.

Die Mahlversuche wurden in einer Schwingmühle mit keramischen Mahlkugeln und -trommeln durchgeführt.

Die eingesetzten Ausgangsmaterialien waren Primaset® PT-30 (Ia, $n \approx 3$), ein gelbes hochviskoses Harz mit $n_D^{98}$ = 1,5667 und einer Gelzeit von 2,5 h, sowie Primaset® CT-90 (Ib, *n* = 1-10), ein gelbes amorphes Pulver mit einer Gelzeit von 0,5 h. Beide Produkte sind bei Lonza AG, Basel, Schweiz, erhältlich.

**Vergleichsbeispiel 1**

**Prepolymere aus Primaset® PT-30**

Primaset® PT-30 wurde zur Verfüssigung 30-40 min auf 120 ˚C erwärmt und in Portionen von jeweils 10-20 g in Einweg-Aluminiumschalen (Ø = 60 mm) gegossen. Die Prepolymerisation wurde bei 165 ˚C für Zeiten von 1-9 h durchgeführt. Die wesentlichen Eigenschaften der so erhaltenen Proben sind in der nachstehenden Tabelle 1 zusammengefasst.

Es zeigte sich, dass mit Primaset® PT-30 allein die gewünschten Eigenschaften nicht erreichbar sind: Entweder ist das Prepolymer nicht mahlbar bzw. zusammenklebend oder es ist schon so weit vernetzt, dass eine Weiterverarbeitung nicht mehr möglich ist.

Tabelle 1

| ***t*** **[h]** | $n_D^{98}$ | **Gelzeit [min]** | **Aspekt (RT)** | **Aspekt (165 ˚C)** | **Mahlbark.** | **Rieselfähigk.** | **Rieselfähigk. nach 3'/50 ˚C** |
|---|---|---|---|---|---|---|---|
| 1 | 1,5695 | - | gb-rt, honigartig | dünnflüssig | - | - | - |
| 2 | 1,5714 | - | gb-rt, honigartig | dünnflüssig | - | - | - |
| 3 | 1,5738 | - | gb-rt, honigartig | dünnflüssig | - | - | - |
| 4 | 1,5744 | - | or-bn, fliesst | dünnflüssig | - | - | - |
| 5 | 1,5780 | - | or-bn, fliesst | dünnflüssig | - | - | - |
| 5½ | 1,5809 | 24 | or-bn, fest, plastisch | dünnflüssig | - | - | - |
| 6 | 1,5810 | 19 | or-bn, fest, plastisch | flüssig | - | - | - |
| 6½ | 1,5846 | 14 | or-bn, fest, plastisch | flüssig | schlecht (mit Trockeneis verrieben) | keine | komplett aufgeschmolzen |
| 7 | 1,588* | 7 | or-bn, fest | zähflüssig | mahlbar | mit Klopfen | klebt zusammen |
| 7½ | nicht | | or-bn, fest | zähflüssig | gut mahlbar | gut | klebt zusammen |
| 8 | bestimmbar | | or-bn, fest | fest | gut mahlbar | gut | nach Klopfen gut |
| 8½ | (lässt sich nicht | | or-bn, fest | fest | gut mahlbar | gut | nach Klopfen gut |
| 9 | verflüssigen) | | or-bn, fest | fest | n. b. | n. b. | n. b. |

***** kaum bestimmbar
gb = gelb; or = orange; rt = rot; bn = braun; n.b. = nicht bestimmt

**Vergleichsbeispiel 2**

**Prepolymere aus Gemischen von Primaset® PT-30 und Primaset® CT-90** (gemeinsame Prepolymerisation)

Es wurde verfahren wie in Vergleichsbeispiel 1 beschrieben, jedoch wurden anstelle von reinem Primaset® PT-30 Gemische aus Primaset® PT-30 und Primaset® CT-90 in den Verhältnissen 90:10, 70:30, 50:50, 30:70, 20:80 und 10:90 eingesetzt. Auch die so erhältlichen Prepolymere waren entweder nicht mahlbar bzw. rieselfähig oder bereits zu stark vernetzt.

**Beispiel 1** (erfindungsgemäss)

Es wurde zunächst verfahren wie in Vergleichsbeispiel 1 beschrieben, jedoch wurden die Proben nach der Prepolymerisation bei 165 ˚C auf 120 ˚C abgekühlt und bei dieser Temperatur mir Primaset® CT-90 vermischt, so dass sich jeweils eine flüssige homogene Mischung bildete. Von diesen Mischungen wurde der Brechungsindex und nach dem Abkühlen auf Normaltemperatur die Mahlbarkeit bestimmt. Ausserdem wurde die Gelzeit gemessen. Gute Ergebnisse wurden bei Prepolymerisationszeiten von 6¼ und 6½ h erhalten. Die wesentlichen Ergebnisse sind in der nachstehenden Tabelle 2 zusammengefasst.

Tabelle 2

| **Ib (Gew.-Teile** | ***t*** **[h]** | **Gelzeit [min]** | **Aspekt (RT/165 ˚C)** | **Mahlbarkeit bei RT** | **Rieselfähigkeit bei RT** |
|---|---|---|---|---|---|
| 0*) | 6¼ | 14 | fest/dickflüssig | - | - |
| 10 | 6¼ | 12 | fest/dickflüssig | gut | gut |
| 20 | 6¼ | 14 | fest/dickflüssig | gut | gut |
| 30 | 6¼ | 16 | fest/dickflüssig | gut | gut |

(fortgesetzt)

| Ib (Gew.-Teile | *t* [h] | Gelzeit [min] | Aspekt (RT/165 °C) | Mahlbarkeit bei RT | Rieselfähigkeit bei RT |
|---|---|---|---|---|---|
| 50 | 6¼ | 14 | fest/dickflüssig | gut | gut |
| 0*) | 6½ | 15 | fest/dickflüssig | - | - |
| 10 | 6½ | 16 | fest/dickflüssig | bedingt | schlecht, mit Klopfen |
| 20 | 6½ | 18 | fest/dickflüssig | gut | schlecht, mit Klopfen |
| 30 | 6½ | 17 | fest/dickflüssig | gut | gut |
| 50 | 6½ | 15 | fest/dickflüssig | gut | gut |
| *) nicht erfindungsgemäss | | | | | |

Die Proben wiesen das gewünschte Temperaturverhalten (fest bei Raumtemperatur, dickflüssig bei 165 °C) und eine vorteilhafte Gelzeit auf. Im Gegensatz zu den nicht erfindungsgemässen Proben (0 Teile Primaset® CT-90) waren alle mahlbar und nach dem Mahlen rieselfähig. Durch Zugabe von 1% hochdisperser Kieselsäure (Aerosil® R972, Degussa-Hüls) konnte die Rieselfähigkeit noch signifikant verbessert werden.

## Patentansprüche

1. Prepolymer-Zusammensetzungen auf Basis von Novolak-Cyanaten der allgemeinen Formel

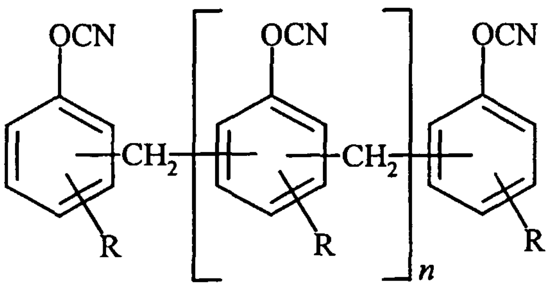

(I),

worin *n* eine Zahl von 0 bis 20 ist und die Reste R gleich oder verschieden sind und für Wasserstoff oder Methyl stehen, **dadurch gekennzeichnet, dass** sie
30 bis 90, vorzugsweise 50 bis 70 Gewichtsteile eines Prepolymeren aus Novolak-Cyanaten (I) mit R = Wasserstoff (Ia) und
10 bis 70, vorzugsweise 30 bis 50 Gewichtsteile Novolak-Cyanate (I) mit R = Methyl (Ib) enthalten.

2. Prepolymer-Zusammensetzungen nach Anspruch 1, **dadurch gekennzeichnet, dass** der Mittelwert von *n* in dem Prepolymer (Ia) 0 bis 5 beträgt.

3. Prepolymer-Zusammensetzungen nach Anspruch 2, **dadurch gekennzeichnet, dass** das eingesetzte Prepolymer des Novolak-Cyanats (Ia) einen Brechungsindex $n_D^{98}$ von mindestens 1,58, vorzugsweise mindestens 1,582 aufweist.

4. Prepolymer-Zusammensetzungen nach einem der Ansprüche 1 bis 3, **dadurch gekennzeichnet, dass** der Mittelwert von *n* in den Novolak-Cyanaten (Ib) 1 bis 10 beträgt.

5. Prepolymer-Zusammensetzungen nach einem der Ansprüche 1 bis 4, **dadurch gekennzeichnet, dass** sie zusätzlich bis zu 5 Gew% eines hochdispersen Siliciumdioxids enthalten.

6. Prepolymer-Zusammensetzungen nach Anspruch 5, **dadurch gekennzeichnet, dass** sie als hochdisperses Siliciumdioxid ein pyrogenes Siliciumdioxid enthalten.

**7.** Prepolymer-Zusammensetzungen nach einem der Ansprüche 1 bis 4, **dadurch gekennzeichnet, dass** sie zusätzlich wenigstens einen partikel- und/oder faserförmigen Füllstoff enthalten.

**8.** Verwendung der Prepolymer-Zusammensetzungen gemäss Ansprüchen 1 bis 7 als Harzkomponente in Basismaterialien für Leiterplatten oder kunstharzgebundenen Schleifkörpern.

## Claims

**1.** Prepolymer compositions based on novolak cyanates of the general formula

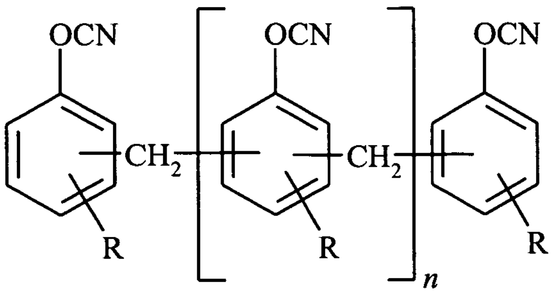

(I),

in which *n* is a number from 0 to 20 and the radicals R are identical or different and represent hydrogen or methyl, **characterized in that** they contain
from 30 to 90, preferably from 50 to 70 parts by weight of a prepolymer of novolak cyanates (I) with R = hydrogen (Ia) and
from 10 to 70, preferably from 30 to 50 parts by weight of novolak cyanates (I) with R = methyl (Ib).

**2.** Prepolymer compositions of claim 1, **characterized in that** the average value of *n* in the prepolymer (Ia) is from 0 to 5.

**3.** Prepolymer compositions of claim 2, **characterized in that** the prepolymer of the novolak cyanate (Ia) used has a refractive index $n_D^{98}$ of at least 1.58, preferably at least 1.582.

**4.** Prepolymer compositions of one of claims 1 to 3, **characterized in that** the average value of *n* in the novolak cyanates (Ib) is from 1 to 10.

**5.** Prepolymer compositions of one of claims 1 to 4, **characterized in that** they further contain up to 5% by weight of a highly disperse silica.

**6.** Prepolymer compositions of claim 5, **characterized in that** as highly disperse silica they comprise a pyrogenic silica.

**7.** Prepolymer compositions of one of claims 1 to 4, **characterized in that** they further comprise at least one particulate and/or fibrous filler.

**8.** Use of the prepolymer compositions of claims 1 to 7 as a resin component in base materials for printed circuit boards or synthetic resin-bound abrasive products.

## Revendications

**1.** Compositions de pré-polymères à base de novolaque-cyanates de formule générale

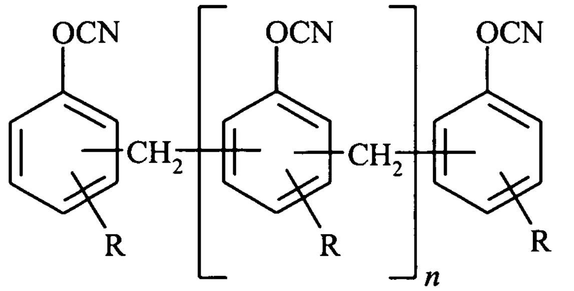

(I),

dans laquelle *n* est un nombre de 0 à 20 et les radicaux R sont identiques ou différents et représentent un atome d'hydrogène ou un groupe méthyle, **caractérisées en ce qu'**elles contiennent :

de 30 à 90, de préférence de 50 à 70 parties en poids d'un pré-polymère de novolaque-cyanates (I), dans laquelle R est un atome d'hydrogène (Ia) et
de 10 à 70, de préférence de 30 à 50 parties en poids de novolaque-cyanates (I), dans laquelle R est un groupe méthyle (Ib).

**2.** Compositions de pré-polymères selon la revendication 1, **caractérisées en ce que** la valeur moyenne de *n* dans le pré-polymère (Ia) est de 0 à 5.

**3.** Compositions de pré-polymères selon la revendication 2, **caractérisées en ce que** le pré-polymère du novolaque-cyanate (Ia) utilisé présente un indice de réfraction $n_D^{98}$ d'au moins 1,58, de préférence d'au moins 1,582.

**4.** Compositions de pré-polymères selon l'une des revendications 1 à 3, **caractérisées en ce que** la valeur moyenne de *n* dans les novolaque-cyanates (Ib) est de 1 à 10.

**5.** Compositions de pré-polymères selon l'une des revendications 1 à 4, **caractérisées en ce qu'**elles contiennent, en outre, jusqu'à 5 % en poids d'une silice hautement dispersée.

**6.** Compositions de pré-polymères selon la revendication 5, **caractérisées en ce qu'**elles comprennent une silice pyrogène en tant que silice hautement dispersée.

**7.** Compositions de pré-polymères selon l'une des revendications 1 à 4, **caractérisées en ce qu'**elles comprennent, en outre, au moins une charge particulaire et/ou fibreuse.

**8.** Utilisation des compositions de pré-polymères selon les revendications 1 à 7, en tant que composant résine dans des matières de base pour des cartes de circuits imprimés ou des produits abrasifs liés par des résines synthétiques.